# EUROPÄISCHE PATENTSCHRIFT

(11) **EP 0 710 480 B1**
(45) Veröffentlichungstag und Bekanntmachung des Hinweises auf die Patenterteilung: **27.05.1998**
(21) Anmeldenummer: 95114318.9
(22) Anmeldetag: 12.09.1995
(51) Int. Cl.: A61K 31/19

(54) **Arzneimittelszusammensetzung aus S-Milchsäure und dessen Verwendung**
Pharmaceutical composition comprising L-lactic acid and its use
Composition pharmaceutique contenant L-acide lactique et son utilisation

(30) Priorität: 12.09.1994 DE 4432429
(43) Veröffentlichungstag der Anmeldung: 08.05.1996
(73) Patentinhaber: Prahm, Heinz, 26817 Rhauderfehn (DE)
(72) Erfinder: Prahm, Heinz, 26817 Rhauderfehn (DE)
(74) Vertreter: Müller-Boré & Partner Patentanwälte

(56) Entgegenhaltungen:
- EP-A- 0 069 291
- EP-A- 0 673 647
- DE-A- 2 721 014
- DE-A- 4 138 896
- FR-A- 2 694 196
- US-A- 4 963 486

## Beschreibung

Die Erfindung betrifft eine Arzneimittelzusammensetzung aus S-Milchsäure und dessen Verwendung für verschiedene Indikationen.

Es gibt drei Modifikationen der Milchsäure, nämlich die physiologische, rechtsdrehende S-Milchsäure (früher auch L(+) genannt), die toxische, linksdrehende R-Milchsäure (früher D(-) genannt) und die racemische S/R-Milchsäure, die sich im Tierkörper verschieden verhalten. Dies gilt auch für den menschlichen Körper. Die S-Milchsäure ist die physiologische Milchsäure, die der Körper selbst in den Muskeln in ausreichender Menge produzieren kann. Sie entsteht hauptsächlich in den Muskelzellen durch Glykolyse. Außerdem wird bei einem gesunden Menschen, der sich schon langfristig biologisch ernährt und dabei eine intakte Darmflora besitzt, im Darmtrakt durch Lactobakterien genügend physiologische S-Milchsäure produziert. Die R-Milchsäure dagegen ist eine Gärungsmilchsäure und stellt ein Zellgift dar, das die Zellfäulnis und Zellwucherung unterstützt. Die R-Milchsäure entsteht im ungesund ernährten Körper durch Gärungsprozesse im Darm. Die Ursache dieser anaeroben Gärung ist die Ernährung mit chemisch behandelten Kohlehydrat-Konzentraten (Süßigkeiten, Schokolade), fetten Kartoffelprodukten (Pommes-frites, Chips), Mastfleisch und stoffwechselbelastenden Fetten. Dadurch kommt es zu einer Veränderung der gesunden Darmflora und es treten schwere toxische Gärungs-und pathogene Fäulnisprozeße im Magen-Darm-Trakt mit toxischer R-Milchsäureproduktion und Phosphorsäure-Übersäuerung auf. Es kommt zu einer pathogenen Übersäuerung des gesamten Organismus, insbesondere des Blut-und Lymphsystems und dadurch ist ein idealer Nährboden für alle möglichen Hefen, Pilze, Viren und Bakterien geschaffen worden.

Bei dem oben erwähnten Racemat handelt es sich um eine Mischung zu gleichen Teilen von spiegelbildlich stereoisomeren Verbindungen (R/S-Milchsäure), das optisch inaktiv ist. Dieses Racemat ist für den Körper weitaus "verträglicher" als die R-Milchsäure allein. Sobald die R-Milchsäure überwiegt und nicht genügend S-Milchsäure durch den Körper gebildet wird, um das Racemat zu bilden, wird der Körper durch die pathogenen Gärungsvorgänge im Körper belastet. Es kommt zu Dyscrasie, Veränderung der Blutgerinnung und zu einer Forcierung des gesamten Krankheitsgeschehens, während bei einem Überangebot an aktiver, physiologischer S-Milchsäure der Körper den Sauerstoff besser auswerten kann und Heilungsprozeße verstärkt werden.

S-Milchsäure wurde schon früher in Arzneimitteln angewendet. So sei auf die DE-C-27 21 014 verweisen, wo ein Echinacea und Milchsäurederivate enthaltendes Kombinationspräparat beschrieben wird, das bei Allergien wirksam ist. R/S-Milchsäure wird bei einer Lösung für die Hautbehandlung und in einer damit hergestellten Naßwindel verwendet, die in DE-OS-26 00 498 beschrieben ist.

Die Aufgabe der Erfindung besteht deshalb darin, ein Arzneimittel bereitzustellen, das die im Körper gebildete R-Milchsäure unschädlich macht und das die durch R-Milchsäure-Überangebot verursachten Krankheiten heilt.

Es wurde nun überraschenderweise festgestellt, daß die übermäßig im ungesund ernährten Körper auftretende R-Milchsäure durch Gabe eines Arzneimittels, das aus einer wäßrigen Lösung von S-Milchsäure und/oder eines ihrer pharmazeutisch verträglichen Salze besteht, "neutralisiert" werden kann und das Racemat R/S-Milchsäure entsteht.

Dieses Unschädlichmachen der R-Milchsäure wirkt sich bei Säugern (Menschen und Tieren) positiv auf verschiedene Erkrankungen aus, wie z.B. Störungen des Blut-, Lymph-, oder Liquorsystems. Außerdem hat sich die erfindungsgemäße Arzneimittelzusammensetzung zur Behandlung von Mykosen und zur Blutgerinnungshemmung bewährt.

Die erfindungsgemäße Arzneimittelzusammensetzung besteht aus einer wäßrigen Lösung von 20-30 Gew.-%, bevorzugt unter 25 Gew.-%, ganz bevorzugt 24 Gew.-%, S-Milchsäure und/oder eines ihrer pharmazeutisch verträglichen Salze. Unter einer wäßrigen Lösung wird erfindungsgemäß das Auflösen der Milchsäure in Wasser oder physiologischer Kochsalzlösung verstanden. Die pharmazeutisch verträglichen Salze sind die Natrium-, Kalium-, Magnesium-, Calcium-, Ammonium-, oder quaternären Ammoniumsalze der S-Milchsäure.

Dabei wird S-Milchsäure und/oder deren pharmazeutisch verträgliche Salze auf die angestrebte Konzentration in Wasser, vorzugsweise physiologischer Kochsalzlösung, gelöst und wird zur Magnetisierung bzw. Polarisierung in ein geeignetes Gefäß gegeben. Unter Verwendung eines geeigneten Geräts, z.B. "Voltcraft DC Power Supply" (Fa. magnet aktiv GmbH), wird die Magnetisierung der Zusammensetzung durchgeführt. Dazu wird eine Goldplatine in die Lösung gegeben und die andere Platine wird am Boden des Gefäßes, in dem sich die Lösung befindet, gelegt. Danach wird über einen Zeitraum von 15-30 Minuten magnetisiert bzw. polarisiert (6V/1A). Erfindungsgemäß ist es auch möglich zuerst eine physiologische Kochsalzlösung zu magnetisieren und dann erst die S-Milchsäure und/oder ihre Salze auf die angestrebte Konzentration zuzusetzen.

Zur Anwendung beim Patienten kommen verdünnte Zubereitungen der erfindungsgemäßen Arzneimittelzusammensetzung. Zu Beginn der Behandlung werden 2-10 Tropfen der 20-30%igen Milchsäurelösung auf 100 ml Wasser verdünnt und vorzugsweise oral verabreicht. Zur Behandlungsfortsetzung kann die Dosis auf 10-20 Tropfen/100 ml Wasser gesteigert werden. Aber auch die percutane, rektale und vaginale Aufnahme von S-Milchsäure-Zubereitungen ist erfindungsgemäß möglich. Schließlich sei auch die Gabe von S-Milchsäure-Verdünnungen per Injektion und Infusion erwähnt. Für die percutane Anwendung sollte die 20-30%ige Milchsäurelösung in Cremes oder Gelzubereitungen auf üblicher Salbengrundlage auf 3-4% verdünnt werden. Eine optimale Dosierung stellt dar, wenn im Blut (und Muskel) 16-25 mg S-Milchsäure/100 ml Vollblut analytisch nachweisbar sind. Bei Überdosierung kommt es zu Muskelkater-artigen Schmerzen, die jedoch nicht gesundheitsschädigend sind.

Das Arzneimittel kann weiter gegebenfalls übliche Arzneimittel-Hilfs-und -Zusatzstoffe enthalten. So wird der Fachmann abhängig von der Verabreichungsform für Osmolarität sorgen, indem er die Arzneimittelzusammensetzung z.B. mit physiologischer Kochsalzlösung, Glucoselösung oder Ringer's Lösung versetzt. Außerdem muß die Arzneimittelzusammensetzung natürlich für die Verabreichung pyrogenfrei sein.

Am Beispiel der Mykosen sei verdeutlicht, daß es unsinnig sein kann, nur den Krankheitserreger, nämlich den Pilz zu bekämpfen, wenn nicht die Ursache für die "Mykosenbereitschaft" beseitigt ist. Bei den Mykosen im und am menschlichen Körper leben die Pilze unter ähnlichen Bedingungen. Sie bevorzugen vorzugsweise feuchtes Gewebe als Wirtsorgan oder Nahrungsspender. Außerdem muß noch eine geschwächte Abwehrlage des Körpers hinzukommen, denn es ist bewiesen, daß bei einer intakten Immunabwehr Mykosen keine Chance haben, sich im oder am Körper festzusetzen. So handeln sich bevorzugt Diabetiker, Menschen nach einer Antibiotika-Therapie und chronisch Kranke als Sekundärkrankheit eine Mykose infolge ihrer Dyscrasie ein. Eine mögliche Theorie ist, daß der Körper die angefallenen Stoffwechselschlacken über Leber und Nieren nicht mehr vollständig ausscheiden kann und er deshalb versucht, die Entgiftung und Ausscheidung vermehrt über die Haut vorzunehmen. Dabei werden die Haut und die Schleimhäute durch starke Konzentrationen von toxischen Säuren und Gasen, einschließlich toxischer R-Milchsäure, pathologisch verändert. Diese Veränderung führt zu einer Bereitschaft für Krankheitserreger einschließlich Pilze empfänglich zu werden und daran zu erkranken. Deshalb nutzt es nur zeitweise, die Mykose mit Antimykotika zu bekämpfen. Nach Absetzen des Medikaments kommt es schnell zu einem Rückfall, da sich an der pathologischen Veränderung der Haut und der Schleimhäute nichts verändert hat und sie weiterhin anfällig für Mykosen bleiben. Deshalb muß durch S-Milchsäure-Gabe dieses ungesunde dyscratische Milieu verändert werden, um die Mykose dauerhaft zu verdrängen.

Auch zur Blutgerinnungshemmung hat sich die Gabe der erfindungsgemäßen Arzneimittelzusammensetzung bewährt. Thromben entstehen u.a. durch eine zu hohe Konzentration von R-Milchsäure im Blut und führen zu dessen Verdickung durch pathogene (R)-Hyperlaktazidosen, die zu einer Erstarrung der roten Blutkörperchen und deshalb zur Gerinnung des Blutes führen. Durch die Agglutination von Blutplättchen, in deren Lücken sich ein lockeres Fibrinnetz abscheidet, in welchem Erythrozyten und Leukozyten liegen, kommt es in strömendem Blut zu Thrombenbildung in den Gefäßen und dem Herzen, so daß Gefäßlumen völlig verschlossen werden können. Durch die Gabe von S-Milchsäure, die die pathologische Wirkung von R-Milchsäure verhindert, wird der Thrombenbildung vorgebeugt bzw. bestehende "Blutverdickung" wird normalisiert.

Es fiel immer wieder auf, daß bei den lokalen Behandlungen von Gichtknoten, Überbeinen und Blutergüssen mit S-Milchsäure per Injektionen und percutan sich diese Krankheitserscheinungen fast momentan auflösten, oder zumindest sich stark verminderten. Bei zum Teil sehr starken entzündlichen Krampfaderbeschwerden und Blutergüssen wurden in 29 Fällen alle sichtbaren und schmerzenden Beschwerden durch S-Milchsäure-Injektionen und Einreibungen so beeinflußt, daß sogar die Verhärtungen und Knotenbildungen nicht mehr fühlbar waren. In 5 Fällen konnte auf verordnete Gummi- und Stützstrümpfe verzichtet werden.

In Laborversuchen konnte noch nachgewiesen werden, daß durch die S-Milchsäure die Erstarrung der roten Blutkörperchen, die Verdickung des Blutes und die Blutgerinnung verhindert werden können und daß sogar die bereits eingetretene Blutgerinnung wieder rückgängig gemacht werden kann.

Die Erfindung wird nun anhand der Beispiele erläutert.

### Beispiel I

Eine Patientin (34 Jahre) kam mit der Diagnose Rheuma/PcP in die Behandlung. Sie war in führenden Kliniken untersucht worden und überall hieß es "Verschleiß-und Abnutzungserscheinungen" mit der Aussicht auf ein baldiges Dasein im Rollstuhl. Diese Diagnose stimmte aber nicht, sondern es handelte sich um eine Stoffwechselentgleisung, die zu einer Dyscrasie und zu einer toxischen R-Milchsäurebildung mit Harnsäureablagerungen an den Gelenken und Muskeln und zu Lymphstauungen, Verhärtungen und Gelenkverformungen und damit zu zeitweise sehr starken Schmerzen führe.

Der Patientin wurden Verdünnungen einer Lösung von 24 Gew-% S-Milchsäure in magnetisiertem Wasser in Verbindung mit verschiedenen lymphabflußwirksamen Stoffen und nierenabflußunterstützenden Stoffen verabreicht. Außerdem mußte sich die Patientin viel bewegen und Gymnastik machen, damit die Muskeln zur S-Milchsäurebildung angeregt wurden. Die Behandlung sah wie folgt aus:

Der Patientin wurden 3 mal täglich 2-10 Tropfen der 24%igen Milchsäurelösung/100 ml Wasser vor und nach dem Essen verordnet und in den ersten 8 Wochen wurde soviel einer Verdünnung der jeweils 24 Gew.-% Milchsäurelösung in magnetisiertem Wasser i.m und s.c. an die betroffenen Gelenke injiziert, gründlich einmassiert und danach Ozon i.m. injiziert, daß im Blut 16-25 mg S-Milchsäure/100 ml Vollblut nachweisbar waren.

Nach 5 Monaten war die Patientin fast beschwerdefrei und konnte wieder ihr Auto lenken. Auch nach über einem Jahr ist ihr Zustand stabil geblieben und die Beschwerden sind verschwunden.

### Beispiel II

Eine Patientin (70 Jahre) hatte jahrelang Dyscrasie, Multiple Sklerose und Gelenkbeschwerden und war nach einem schweren Unfall an beiden Händen wegen Trümmerbrüchen operiert worden und bekam darauf täglich Eispackungen an beiden Händen mit anschließenden krankengymnastischen Übungen verordnet, mit dem Erfolg, daß die Hände wieder dünn und beweglich wurden. 15 Monate nach dem Unfall mußte die Kältetherapie abgebrochen werden, da sie plötzlich starke Hüftgelenkbeschwerden bekam. Außerdem wurde aufgrund plötzlich auftretender Magen-/Darmbeschwerden und Lymphknotenverhärtungen Darmkrebs diagnostiziert, woran sie sofort operiert wurde.

Nach der Operation wurden der Patientin 5 mal täglich je 50 Tropfen einer Lösung von 24 Gew.-% S-Milchsäure in magnetisierter Kochsalzlösung vor und nach jedem Essen verabreicht und es wurden 2 mal täglich Injektionen i.m. je 25 ml und 2 mal täglich Darmklistiere mit je 25 ml einer Verdünnung der 24%igen S-Milchsäurelösung durchgeführt, so daß im Blut ca. 16-25 mg Milchsäure/100 ml Blut nachweisbar waren.

Der Patientin ging es zusehends besser und die Lymphdrüsen- und Gelenkverhärtungen machten keine Beschwerden mehr. Es wurden die Blutwerte vor der Operation und 3 Monate nach der Operation bestimmt:

| | | |
|---|---|---|
| Hb: | 10,7 vor der Op. | 12,0 3 Mon. nach der Op. |
| Erythrozyten | 3,87 | 4,05 |
| Leukozyten | 4,9 | 9,8 |
| Thrombozyten | 497 | 361 |
| Calcium | 3,9 | 2,2 |

Auffallend ist hier der durch die Therapie mit S-Milchsäure zu sehende Thrombozytenabbau und die sichtbaren Veränderungen an den Lymphdrüsen und Gelenken.

### Beispiel III

In einem Versuch wurde vom Schlachter 500 ml warmes Schweineblut in einem angewärmten Thermogefäß geholt. Als der Versuch beginnen sollte, hatte sich das Blut bereits abgesetzt und das Bluteiweiß war geronnen. Die Erkenntnis, daß beim Muskelkater bis zu 300 mg% Muskel-Fleischmilchsäure, also physiologische S-Milchsäure gebildet werden können und die Fließgeschwindigkeit des Blutes dabei am größten ist, bildete die Überlegung und den Entschluß, diesem geronnenen Blut 1,5 ml einer Lösung von 30 Gew.-% S-Milchsäure in magnetisiertem Wasser zuzusetzen. Mit einem Kunststofflöffel wurde kräftig gerührt und nach 3 Minuten war das Blut wieder flüssig und die Blutgerinnung aufgehoben.

In weiteren Versuchsreihen mit tierischem Blut wurde jeweils 10 ml Blut 1.) = 1,5 mg; 2.) = 2,5 mg; 3.) = 5 mg; 4.) = 10 mg; 5.) = 20 mg; und 6.) = 30 mg feste S-Milchsäure zugesetzt, kräftig geschüttelt und anschließend in einem elektrischen Warmhaltegerät die Wassertemperatur konstant auf 36°C gehalten. Das Blut bildete keine Gerinnung, es blieb flüssig. Nach gut einer Stunde fing die Probe 1, und danach nach und nach auch die anderen Proben an zu gelieren und fest zu werden (keine Gerinnung).

## Patentansprüche

1. Arzneimittelzusammensetzung bestehend aus einer wäßrigen Lösung von 20-30 Gew.-% S-Milchsäure und/oder deren pharmazeutisch verträglichen Salzen und gegebenenfalls üblichen Arzneimittel-Hilfs- und -Zusatzstoffen.

2. Arzneimittelzusammensetzung nach Anspruch 1, wobei es sich bei der wäßrigen Lösung um S-Milchsäure in physiologischer Kochsalzlösung handelt.

3. Arzneimittelzusammensetzung nach Anspruch 1 oder 2, wobei die Zusammensetzung einer Magnetisierung bzw. Polarisierung unterworfen wird.

4. Arzneimittelzusammensetzung nach einem der Ansprüche 1-3, wobei diese oral, percutan, rektal, vaginal oder durch Injektion oder Infusion verabreichbar ist.

5. Arzneimittelzusammensetzung nach einem der Ansprüche 1-4, wobei die Lösung 24 Gew.-% Milchsäure in physiologischer Kochsalzlösung ist.

6. Verwendung der Arzneimittelzusammensetzung nach einem der Ansprüche 1-5 zur Herstellung einer einem Säuger verabreichbaren Zubereitung für die Therapie von Störungen des Blut-, Lymph-, oder Liquorsystems, zur Blutgerinnungshemmung sowie zur Therapie von Mykosen.

## Claims

1. Pharmaceutical composition consisting of an aqueous solution of 20-30 %, by weight, of S-lactic acid and/or its pharmaceutically acceptable salts and optionally usual pharmaceutical auxiliary substances or pharmaceutical additives.

2. Pharmaceutical composition according to claim 1, wherein the aqueous solution is S-lactic acid in a physiological saline solution.

3. Pharmaceutical composition according to claim 1 or 2, wherein the composition is subjected to magnetisation or polarisation.

4. Pharmaceutical composition according to any of claims 1 to 3, wherein the composition is applicable oral, percutaneous, rectal, vaginal, or by injection or by infusion.

5. Pharmaceutical composition according to any of claims 1 to 4, wherein the solution is 24 %, by weight, of lactic acid in a physiological saline solution.

6. Use of the pharmaceutical composition according to any of the claims 1 to 5 for the preparation of a formulation applicable to mammals for the therapy of disorders of the blood system, lymphatic system or liquor system, for the inhibition of blood coagulation and for the therapy of mycosis.

## Revendications

1. Composition pharmaceutique consistant une solution aqueuse ayant une proportion de 20 à 30% en poids d'acide lactique de type S et/ou de ses sels pharmaceutiquement acceptables et le cas échéant des substances pharmaceutiques additives et additionnelles courantes.

2. Composition pharmaceutique selon la revendication 1, caractérisée en ce que la solution aqueuse est de l'acide lactique de type S dissous dans une solution physiologique de sel de table.

3. Composition pharmaceutique selon la revendication 1 ou 2, caractérisée en ce que la composition est soumise à une magnétisation ou une polarisation.

4. Composition pharmaceutique selon l'une quelconque des revendications 1 à 3, caractérisée en ce qu'elle peut être administrée sous forme orale, percutanée, rectale, vaginale ou par injection ou infusion.

5. Composition pharmaceutique selon l'une quelconque des revendications 1 à 4, caractérisée en ce que la solution est une solution physiologique de sel de table comprenant 24% en poids d'acide lactique.

6. Utilisation d'une composition pharmaceutique selon l'une quelconque des revendications 1 à 5 pour la manufacture d'une préparation administrable à un mammifère pour le traitement thérapeutique des troubles du système sanguin, lymphatique ou des fluides, pour l'inhibition de la coagulation du sang, ainsi que pour le traitement thérapeutique des mycoses.
